(19)

Europäisches Patentamt

European Patent Office

Office européen des brevets

(11) **EP 4 520 271 A1**

(12) **EUROPEAN PATENT APPLICATION**

(43) Date of publication:
**12.03.2025 Bulletin 2025/11**

(21) Application number: **23196543.5**

(22) Date of filing: **11.09.2023**

(51) International Patent Classification (IPC):
**A61B 8/04** (2006.01)   **A61B 8/06** (2006.01)
**A61B 8/08** (2006.01)   **A61B 5/02** (2006.01)
**A61B 8/00** (2006.01)

(52) Cooperative Patent Classification (CPC):
**A61B 8/04; A61B 5/02007; A61B 5/021;
A61B 8/06; A61B 8/0891; A61B 8/486;
A61B 8/488; A61B 8/5223;** A61B 8/54

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **Koninklijke Philips N.V.
5656 AG Eindhoven (NL)**

(72) Inventors:
• **JOSHI, Rohan**
 **Eindhoven (NL)**
• **WIJSHOFF, Ralph Wilhelm Christianus Gemma
 Rosa**
 **Eindhoven (NL)**
• **MENA BENITO, Maria Estrella**
 **5656AG Eindhoven (NL)**

(74) Representative: **Philips Intellectual Property &
Standards
High Tech Campus 52
5656 AG Eindhoven (NL)**

(54) **DETERMINING VASCULAR COMPLIANCE**

(57)    Disclosed concepts aim to provide schemes, solutions, concepts, designs, methods, and systems pertaining to a determining vascular compliance of a subject. Specifically, a blood pressure and arterial flow (e.g., from arterial diameter and/or velocity of blood through the artery) of a first and second artery (the same arteries or corresponding arteries on contralateral limbs) are measured. From the blood pressure, a pulse pressure can be determined, and from the arterial flow a pulse volume can be determined. From the pulse pressure and volume pressure, vascular compliance can be determined. Accordingly, a direct and accurate estimation of vascular compliance may be obtained in a non-invasive manner. Also disclosed is the use of the determined vascular compliance in a transfer function to obtain accurate estimation of hemodynamic parameters (e.g., cardiac output and stroke volume) of the subject.

FIG. 4

EP 4 520 271 A1

**Description**

FIELD OF THE INVENTION

The present invention relates to the field of vascular compliance determination.

BACKGROUND OF THE INVENTION

Non-invasive estimation of stroke volume and cardiac output of a subject is typically accomplished by applying transfer functions to physiological parameters which can be conveniently non-invasively measured. Such non-invasive physiological parameters include blood pressure and demographic information (e.g., height, weight, age, and gender) that can be easily obtained from the subject.

Subject demographic information can be used to estimate vascular compliance, which indicates how a measured pulse pressure relates to the underlying pulse volume, as needed for the determination of stroke volume and/or cardiac output. However, basing the estimation of vascular compliance on demographics is often inaccurate as: (i) the vascular age of the subject may be different than the chronological age of the subject; and (ii) the vascular condition is not a constant function of solely demographics but depends on the underlying time-varying subject-specific hemodynamic situation.

Accordingly, the inventors have identified a need for an improved means of determining vascular compliance so as to improve estimation of hemodynamic parameters.

SUMMARY OF THE INVENTION

The invention is defined by the claims.

According to examples in accordance with an aspect of the invention, there is provided a system for determining vascular compliance of a subject.

The system comprises a blood pressure sensor configured to measure blood pressure of a first artery of the subject;

> an ultrasound sensor configured to measure arterial flow of a second artery of the subject, wherein the first artery and the second artery are the same artery or corresponding arteries of contralateral limbs;
> a controller configured to:
>
>> control the blood pressure sensor to measure the blood pressure;
>> control the ultrasound sensor to measure the arterial flow; and
>
> a processor configured to:
>
>> determine a pulse pressure based on the measured blood pressure;
>> determine a pulse volume based on the measured arterial flow; and
>> determine a vascular compliance of the subject based on the pulse pressure and the pulse volume.

Examples of the disclosure are based on the realisation that, by provision of an ultrasound sensor and a blood pressure sensor, vascular compliance of a subject can be directly determined.

An ultrasound sensor can be utilised to determine the arterial flow of an artery (i.e. the rate at which blood flows through an artery). This means that changes in diameter of the artery and/or velocity of the blood flowing through the artery are measured. Indeed, operating an ultrasound sensor in Doppler mode or color Doppler mode enables measurements of the velocity of the blood, and operating the ultrasound sensor in M-mode, B-mode or A-mode enables measurements of the diameter. From this arterial flow information, the volume change in the artery due to a cardiac pulse (i.e. a heartbeat), or pulse volume, may be determined in a variety of ways.

Of course, blood pressure measurements from a blood pressure sensor (e.g., a pressure cuff) may be leveraged to determine the pressure in the artery due to a cardiac pulse (i.e. a heartbeat), or pulse pressure. Typically, this is achieved by determining the difference between systolic and diastolic blood pressure.

Generally, the vascular compliance, C, can be determined from the pulse volume, $\Delta V$, and the pulse pressure, $\Delta P$, according to the following:

$$C = \frac{\Delta V}{\Delta P}. \qquad\qquad [1]$$

It is worth noting that the above is not considered to be limiting. Indeed, this disclosure provides multiple methods that the arterial flow and blood pressure measurements may be used to determine pulse volume and pulse pressure and the corresponding vascular compliance.

In any case, when a vascular compliance of a subject is known, hemodynamic parameters such as cardiac output and stroke volume may be accurately estimated. In contrast to the typical use of demographic information to aid in the prediction of such hemodynamic parameters, the present invention enables a direct determination of vascular compliance that may be accurate in spite of differences between the chronological and vascular ages of the subject, as well as time-varying hemodynamic changes.

The controller may be configured to control the blood pressure sensor to measure the blood pressure, and the ultrasound sensor to measure the arterial flow simultaneously or substantially sequentially. Indeed, it is not a requirement for the blood pressure and arterial flow information to be acquired at precisely the same time. This may actually not be desirable in the case that a pressure cuff is used to measure blood pressure, which may squeeze the dimensions of the artery and alter arterial flow measurements. However, it may be desirable to perform both measurements in close temporal proximity (i.e. substantially sequentially, or one-after-the-other) in order to ensure an accurate vascular compliance determination.

The first artery may be a first brachial artery of the subject. The second artery may be the first brachial artery or a second brachial artery of the contralateral limb of the first brachial artery. Subjects may find that blood pressure and ultrasound measurements on the brachial artery are familiar and comfortable.

In some embodiments, the controller may be configured to control the blood pressure sensor to measure a systolic pressure and a diastolic pressure. In this case, the processor may be configured to determine the pulse pressure based on the systolic pressure and the diastolic pressure. This provides the minimum requirements for the determination of the pulse pressure.

The ultrasound sensor may be configured to measure a velocity of blood through the second artery and a diameter of the second artery. The controller may control the ultrasound sensor to measure the velocity and the diameter, and the processor may be configured to determine the pulse volume based on the velocity and the diameter.

The most accurate and reliable measure for the pulse volume (i.e., change in arterial blood volume as a result of a cardiac pulse) is based on both the velocity of blood through the artery during the cardiac pulse, and the diameter of the artery during the cardiac pulse.

In this case, the ultrasound sensor may be configured to operate using pulsed wave Doppler mode or color Doppler mode to measure the velocity, and to operate using M-mode, B-mode or A-mode to measure the diameter.

Alternatively, the ultrasound sensor may be configured to measure a diameter of the second artery. Thus, the controller may control the ultrasound sensor to measure the diameter, and the processor may be configured to determine the pulse volume based on the diameter.

The diameter of the artery during a cardiac pulse can be used as an estimate for the pulse volume even in absence of a velocity measurement. That is, the change in the diameter of the artery during the pulse will correlate to the change in arterial blood volume as a result of the pulse, and can be used to derive such a pulse volume without requiring the ultrasound sensor to be configured for velocity measurement.

As yet another alternative, the ultrasound sensor may be configured to measure a velocity of blood through the second artery. The controller may control the ultrasound sensor to measure the velocity, and the processor may be configured to determine the pulse volume based on the velocity.

As above, the velocity of the blood travelling through the artery during a cardiac pulse can also be used as an estimate for the pulse volume, even in absence of a diameter measurement. Indeed, the pulse volume may be estimated in this case using an average measured velocity of blood through the artery or a change in average measured velocity of blood through the artery, and an estimate of the arterial diameter. Thus in this embodiment, the pulse volume may be derived without requiring the ultrasound sensor to be configured for diameter measurement.

The controller may be further configured to control the blood pressure sensor to measure a blood pressure signal over at least one heart cycle. In this case, the processor may be configured to determine a time constant of exponential decay of the blood pressure based on a decay of blood pressure following systole in the continuous blood pressure signal; determine a peripheral vascular resistance based on a mean blood pressure over the at least one heart cycle and a mean of the measured arterial flow; and determine the vascular compliance of the subject based on the time constant and the peripheral vascular resistance.

Yet another means for determining the vascular compliance is related to the time constant of exponential decay of the blood pressure between systole and diastole. In this case, the blood pressure measurement is taken continuously throughout the whole cycle to determine the time constant, and a peripheral vascular resistance determined based on the measured blood pressure and the arterial flow measured by the ultrasound monitor. The vascular compliance can thus be determined by the resulting time constant and peripheral vascular resistance.

This is advantageous as it may be achieved by a single measurement of the arterial flow, requiring less hardware and saving cost.

When the first artery and the second artery are the same artery, the blood pressure sensor may be configured to measure blood pressure of the first artery of the subject at a first location, and the ultrasound sensor may be configured to measure arterial flow of the second artery in two longitudinally separated locations of the second artery. The first location may be between the two longitudinally separated locations. In this case, the processor may be configured to determine the vascular compliance of the subject further based on the measured arterial flow in the two longitudinally separated locations.

As a more accurate determination of vascular compliance, the arterial flow may be measured by the ultrasound sensor in two locations, with the blood pressure sensor measuring blood pressure in between.

In some embodiments, the controller may be configured to: control the blood pressure sensor to measure the blood pressure for a first time period; control the ultrasound sensor to measure the arterial flow for the first time period; control the blood pressure sensor to measure the blood pressure for a second time period; and control the ultrasound sensor to measure the arterial flow for the second time period, wherein the second time period and first time period are sequential. In this case, the processor may be configured to: determine the pulse pressure based on a difference between the measured blood pressure in the first time period and the measured blood pressure in the second time period; and determine a pulse volume based on a difference between the measured arterial flow in the first time period and the measured arterial flow in the second time period.

Differences in blood pressure and arterial flow over two subsequent epochs/periods of time can also be used to determine the pressure pulse and volume pulse, and may further improve accuracy of the resultant determined vascular compliance.

The controller may be configured to control the blood pressure sensor to measure the blood pressure over a plurality of heart cycles. The processor may be configured to determine the pulse pressure based on an average of the measured blood pressure over the plurality of heart cycles.

An average of the blood pressure over a plurality of heart cycles, for example a phase rectified signal average (PRSA), may reduce noise in the measured blood pressure and improve the accuracy of determination of the pulse pressure.

Similarly, the controller may be configured to control the ultrasound sensor to measure the arterial flow over a plurality of heartbeats. The processor may be configured to determine the pulse volume based on an average of the arterial flow over the plurality of heart beats.

As for blood pressure, an average over a plurality of heart cycles/heart beats may smooth noise present in individual heart cycles/heart beats. Thus, taking an average, such as a PRSA, may reduce noise in the measured arterial flow, and improve accuracy of determination of pulse volume.

The controller may be configured to continually control the ultrasound sensor to measure the arterial flow. In this case, the processor may be configured to detect a change of the measured arterial flow in time meeting a predetermined condition. Then, the controller may be configured to control the blood pressure sensor to measure the blood pressure responsive to detecting the change of the arterial flow.

That is, the system may be triggered to determine a vascular compliance when there is a change in the arterial flow. Ultrasound measurements taken by an ultrasound sensor are often non-invasive and comfortable for a patient. In contrast, blood pressure measurements can be disturbing with the operation of a pressure cuff or similar. Therefore, only operating the system when a change in arterial flow is determined by the ultrasound sensor may improve subject comfort during long periods of subject monitoring whilst not missing any changes in vascular compliance (reflecting changes in hemodynamic parameters).

According to examples in accordance with another aspect of the invention, there is provided a method of determining vascular compliance of a subject, comprising:

> controlling a blood pressure sensor to measure blood pressure of a first artery of the subject;
> controlling an ultrasound sensor to measure arterial flow of a second artery of the subject, wherein the first artery and the second artery are the same artery or corresponding arteries of contralateral limbs;
> determining a pulse pressure based on the measured blood pressure;
> determining a pulse volume based on the measured arterial flow; and
> determining a vascular compliance of the subject based on the pulse pressure and the pulse volume.

According to further examples in accordance with an aspect of the invention, there is provided a method for determining hemodynamic parameters of a subject, comprising:

> obtaining a blood pressure measurement of a subject;
> determining vascular compliance of the subject according to any embodiments described herein; and
> processing the vascular compliance and the blood pressure measurement of the subject using a transfer function to determine the hemodynamic parameter.

In some embodiments, the method for determining hemodynamic parameters may further comprise obtaining an arterial flow measurement of the subject. In this case, the processing step may include processing the vascular compliance, blood pressure measurement of the subject, and the arterial flow measurement of the subject using the transfer function to determine the hemodynamic parameter.

According to other examples in accordance with a further aspect of the invention, there is provided a computer program comprising computer program code means adapted, when said computer program is run on a computer, to implement the method of determining vascular compliance of a subject and/or determining hemodynamic parameters of a subject.

These and other aspects of the invention will be apparent from and elucidated with reference to the embodiments described hereinafter.

BRIEF DESCRIPTION OF THE DRAWINGS

For a better understanding of the invention, and to show more clearly how it may be carried into effect, reference will now be made, by way of example only, to the accompanying drawings, in which:

Fig. 1 presents a blood pressure waveform acquired over 30s and a graph representing a phase-rectified signal average of the blood pressure waveform;
Fig.2 presents an arterial velocity waveform acquired over 30s and a graph representing a phase-rectified signal average of the velocity waveform;
Fig.3 is a vascular model used to determine vascular compliance;
Fig.4 presents a block diagram of a system for determining vascular compliance of a subject according to an aspect of the invention;
Fig.5 presents a flow diagram of a method for determining vascular compliance of a subject according to another aspect of the invention;
Fig.6 presents a flow diagram of a method for determining a hemodynamic parameter of a subject according to a further aspect of the invention; and
Fig.7 is a simplified block diagram of a computer within which one or more parts of an embodiment may be employed.

DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. The mere fact that certain measures are recited in mutually different dependent claims does not indicate that a combination of these measures cannot be used to advantage.

It should be understood that the Figures are merely schematic and are not drawn to scale. It should also be understood that the same reference numerals are used throughout the Figures to indicate the same or similar parts.

Proposed concepts aim to provide schemes, solutions, concepts, designs, methods, and systems pertaining to a determining vascular compliance of a subject. Specifically, a blood pressure and arterial flow (e.g., from arterial diameter and/or velocity of blood through the artery) of a first and second artery (the same arteries or corresponding arteries on contralateral limbs) are measured. From the blood pressure, a pulse pressure can be determined, and from the arterial flow a pulse volume can be determined. From the pulse pressure and pulse volume, vascular compliance can be determined. Accordingly, a direct and accurate estimation of vascular compliance may be obtained in a non-invasive manner. Also proposed is the use of the determined vascular compliance in a transfer function to obtain accurate estimation of hemodynamic parameters (e.g., cardiac output and stoke volume) of the subject.

In other words, the present disclosure proposes the use of ultrasound and blood pressure measurements to directly assess the vascular compliance of a subject. Vascular compliance indicates how a measured pulse pressure relates to the underlying pulse volume. It has thus been realised that the vascular compliance of a subject can be assessed by measuring the pulse volume ($\Delta V$) and pulse pressure ($\Delta P$) on the same or contralateral arteries of the subject by using an ultrasound sensor and a blood pressure sensor, respectively. Using the resulting measurements, an improved estimate of vascular compliance may be realised. The improved vascular compliance determination/estimation may then be used in transfer functions for improved estimation of stroke volume and cardiac output of a subject, and/or may be fed into pulse contour algorithms.

By way of explanation, hemodynamic monitoring, (e.g., monitoring cardiac output and stroke volume in subjects) is needed for the early detection, identification, and management of life-threatening clinical conditions, such as sepsis and cardiogenic shock. In addition, such monitoring is useful for assessing the efficacy of therapeutic/pharmaceutical interventions such as the administration of vasopressors.

The gold standard for measuring cardiac output is the thermodilution method (e.g., used in a Swan-Ganz pulmonary artery catheter). This is an invasive and intermittent method and can be used several times a day, but not continuously.

One method that offers continuity of hemodynamic measurements is based on pulse contour analysis. This approach utilizes blood pressure measurements, typically measured arterially with an invasive line. A transfer function utilizing the BP waveform along with demographic information of the subject (e.g., height, weight, age, and gender) is used to estimate hemodynamic parameters continuously.

Currently, transfer functions used in pulse contour analysis include demographic information as a proxy measure for estimating vascular compliance. This is because no non-invasive devices exist for measuring vascular compliance directly, rendering measurement of vascular compliance a complex task.

However, demographic model-based estimation of compliance is inaccurate, because:

(i) the vascular age of a subject may be different than the chronological age of the subject due to lifestyle choices, chronic illnesses, and genetic variations; and
(ii) the vascular condition of a subject is not a constant function of solely demographics but depends also on the patient-specific hemodynamic situations (e.g., pharmaceuticals, centralization of cardiac output, etc) which varies over time.

Furthermore, it is known that the accuracy of calibrated pulse contour algorithms falls with time. Thus, a direct measure of vascular compliance may improve the accuracy of both calibrated and uncalibrated pulse contour algorithms.

Accordingly, implementations provide the use of ultrasound and blood pressure measurement to directly determine vascular compliance, and incorporate this determined vascular compliance into transfer functions used in pulse contour analysis.

Specifically, this can be achieved by measuring the pulse volume ($\Delta V$) and the pulse pressure ($\Delta P$) on the same artery or contralateral arteries by using an ultrasound device/sensor and blood pressure device/sensor (e.g., a cuff or an arterial line).

Vascular compliance, C, can be calculated as follows:

$$C = \frac{\Delta V}{\Delta P}, \qquad\qquad [2]$$

with $\Delta V$ being the change in arterial blood volume due to a cardiac pulse, and $\Delta P$ being the corresponding change in arterial blood pressure due to a cardiac pulse (e.g., a difference between the systolic pressure and diastolic pressure, also known as the pulse pressure).

The minimum requirements for implementing the invention are therefore:

(i) a blood pressure sensor (e.g., pressure cuff, arterial line or any other device that generates blood pressure estimates), measurements from which can be used to determine the pulse pressure; and
(ii) an ultrasound sensor that can measure arterial flow. For example, an ultrasound sensor that can be used to measure arterial lumen diameter and/or arterial velocity can then be used to estimate arterial blood flow. Measurements from the ultrasound sensor can then be used to determine the pulse volume.

Preferably, the blood pressure measurement and arterial flow measurement may be conducted by the blood pressure sensor and ultrasound sensor in close physical proximity, e.g., a short distance apart on an arterial branch like the brachial artery. This ensures an accurate estimate of the local compliance. Nonetheless, it is possible to measure blood pressure and arterial flow in different locations, for instance in contralateral limbs of the subject.

Pulse volume and pulse pressure (from which vascular compliance may be derived) can be determined from blood pressure sensor and ultrasound sensor measurements in many ways. Some of these methods will be outlined below.

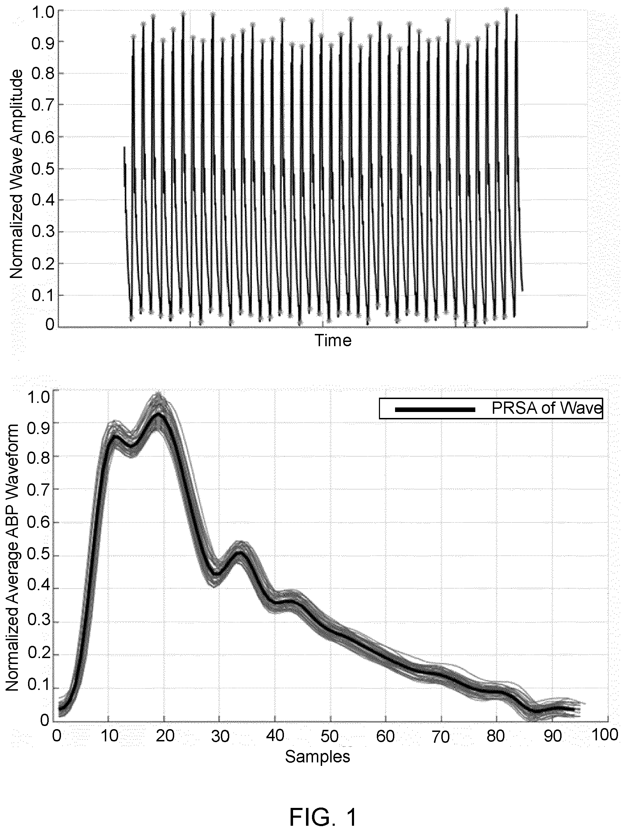

Fig. 1 presents (top graph) an arterial blood pressure waveform acquired over a 30s epoch, as well as (bottom graph) a graph representing a phase-rectified signal average (PRSA) of the arterial blood pressure waveform to create an ensemble average blood pressure waveform.

The pulse pressure of a subject can be measured over a single beat by simply subtracting the diastolic pressure from the systolic pressure as measured by a blood pressure sensor. However, this approach may be noisy (e.g., due to respiration or movement). To capture a representative measure of pulse pressure, and therefore vascular compliance, multiple heart beats of the blood pressure waveform can be averaged to create a PRSA (with e.g. the peaks serving as an anchor point) over multiple beats. This is shown in Fig. 1. The pulse pressure of this ensemble average can serve as a more robust measure of ΔP.

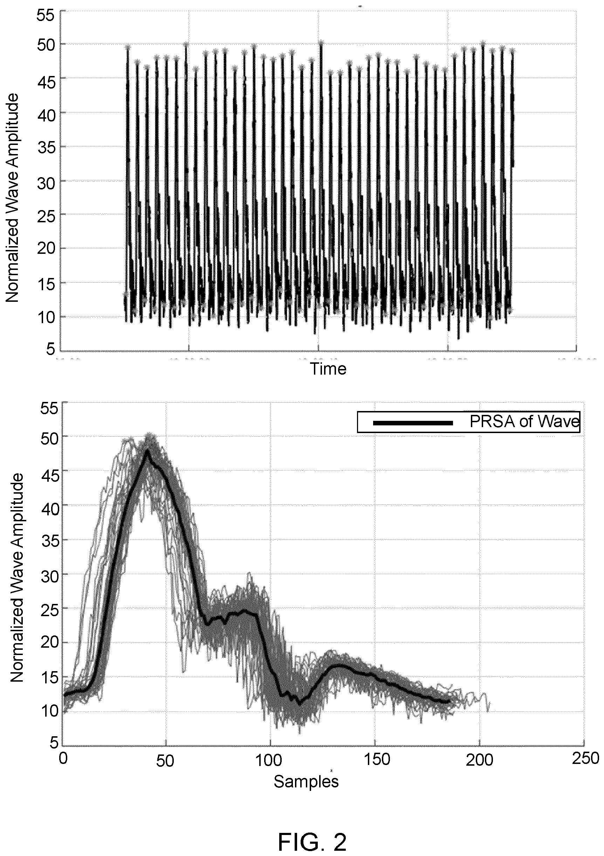

Fig.2 presents (top graph) an arterial velocity waveform acquired over a 30s epoch, as well as (bottom graph) a graph representing a PRSA of the velocity waveform to create a representative velocity beat.

As shown, an ensemble average of the velocity waveform (corresponding to a heartbeat) can be extracted. This velocity waveform can be multiplied by the average artery cross section (i.e., π×((average diameter)/2)^2) over the epoch to acquire an estimation of the arterial flow in volume per second.

For instance, the diameter can be extracted by using an ultrasound sensor operating in M-mode, B-mode, or A-mode. The velocity can be extracted by using an ultrasound sensor operating using pulsed wave Doppler or color Doppler.

Thus, the ensemble-averaged pulse pressure and pulse velocity may be measured and used to obtain an estimate of a vascular compliance of an artery (e.g., a brachial artery) of a subject. The following steps could be employed:

(i) The vascular compliance of the artery can be determined as C = ΔV/ΔP, with ΔV (e.g., in cm$^3$) the change in arterial blood volume due to a cardiac pulse and ΔP (e.g., in mmHg) the corresponding change in arterial blood pressure due to a cardiac pulse;

(ii) The averaged measure of pulse pressure (ΔP) within an epoch can be measured, for instance by calculating ΔP as the difference between the systolic and diastolic pressure levels of the ensemble-averaged pulse pressure shown in Fig. 1;

(iii) Arterial flow volume may be calculated by multiplying the velocity by the average artery cross-section, and volume can be obtained by integration of the flow over time. Integration of the arterial flow provides an estimate of the blood volume that traversed through the artery in a longitudinal direction. To obtain arterial blood volume change (i.e. pulse volume) the integral of the flow over the entire pulse duration may be used, or the integral of the flow from the diastolic to the systolic time instants may be used;

(iv) Even though the pulse volume that is determined in this way is moving in the longitudinal direction in the artery, the pulse volume does reflect vascular compliance because of the change in arterial lumen. In fact, even the shape of the pressure wave gets affected by the vascular compliance, thus allowing the use of Equation 1 to estimate arterial compliance;

(v) Alternatively, ΔP and ΔV may be measured by measuring relative changes over two subsequent epochs, each e.g. 30 s long, where ΔP can be determined as the difference between the pressures (e.g., mean, systolic or diastolic) obtained for the individual epochs, and ΔV as the difference between the longitudinal volumes obtained for the individual epochs.

As another option for determining the vascular compliance using blood pressure and ultrasound measurements, the distention of the artery in a radial direction as a result of the cardiac pressure pulse needs to be understood. The simplest estimate of vascular compliance can be based on the pressure pulse ΔP and the resulting diameter change (ΔD), i.e., $D_{systole}$ - $D_{diastole}$ of the artery, with $D_{systole}$ the artery diameter at systole and $D_{diastole}$ the brachial artery diameter at diastole. In this case an estimate of vascular compliance can be obtained as ΔD/ΔP [mm/mmHg].

The change in artery diameter is preferably not measured during a cuff inflation of a blood pressure sensor, as that changes the shape of the artery. In this embodiment, changes in volume of arterial flow are not measured, and thus the ultrasound sensor may operate in only M-mode, B-mode or A-mode to give measures of change in diameter of the artery (i.e., as a proxy for arterial flow).

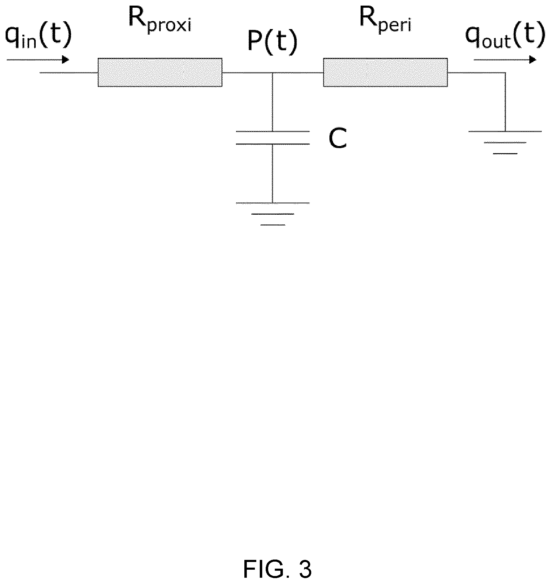

Alternatively, Fig. 3 presents a vascular model that may be used to determine vascular compliance, C, from arterial/blood pressure and one or two arterial flow measurements. Specifically, $q_{in}$(t) represents an arterial flow into the artery (i.e., upstream from a blood pressure sensor), $q_{out}$(t) an arterial flow out of the artery (i.e., downstream from a blood pressure sensor), and P(t) is a measured blood pressure. $R_{proxi}$ and $R_{peri}$ represent vascular resistance proximate to the blood pressure measurement and distal to the blood pressure measurement, respectively.

In essence, Fig. 3 represents that the vascular compliance can also be assessed from the exponential decay in the blood pressure following systole. In this phase of the pressure pulse, starting from the systolic pressure at the systolic time instant $t_{sys}$, one can fit an exponential decay according to:

$$P(t) = (P_{sys} - P_{dia})e^{-\frac{t-t_{sys}}{\tau}} + P_{dia} = (P_{sys} - P_{dia})e^{-\frac{t-t_{sys}}{R_{peri}C}} + P_{dia}, \qquad [3]$$

$$for \quad t \in [t_{sys}, t_{dia}]$$

with $P_{sys}$=P($t_{sys}$) being the systolic pressure at the systolic time instant $t_{sys}$, $P_{dia}$=P($t_{dia}$) being the diastolic pressure at the diastolic time instant $t_{dia}$, τ=$R_{peri}$C being the time constant of the exponential decay, $R_{peri}$ being the peripheral vascular resistance, and C being the vascular compliance. The exponential fit provides an estimate of the time constant $\tau$ which is the product of peripheral vascular resistance and the vascular compliance. The peripheral vascular resistance can be estimated from the arterial pressure and the arterial flow. The arterial flow can be obtained by multiplying the (average) arterial flow velocity in Fig.2 by the average cross section of the artery. An estimate of the peripheral vascular resistance can be obtained as the ratio of the mean of the ensemble-averaged pressure pulse, and the mean of the ensemble-averaged flow pulse. This can be represented as:

$$R_{peri} = \frac{\frac{1}{T_{pulse}} \int_0^{T_{pulse}} P(t)dt}{\frac{1}{T_{pulse}} \int_0^{T_{pulse}} q_{out}(t)dt} = \frac{MAP}{MAF}, \qquad [4]$$

with $T_{pulse}$ being the duration of the ensemble-averaged pulse, MAP being the mean arterial pressure, and MAF being the mean arterial flow. The estimate of the brachial artery compliance can then be obtained as

$$C = \frac{\tau}{R_{peri}}. \qquad [5]$$

A more advanced measurement of vascular compliance can be based on two arterial flow measurements ($q_{in}$ and $q_{out}$) as illustrated in Fig. 3. Generally, arterial flow can be obtained by multiplying the velocity by the average cross section of the artery. In this case, the relationship between the artery/blood pressure, and the arterial flows through the brachial artery can be expressed as follows:

$$C \frac{dP(t)}{dt} = q_{in}(t) - q_{out}(t), \qquad [6]$$

or

$$P(t_1) - P(t_0) = \frac{1}{C} \int_{t_0}^{t_1} \big(q_{in}(t) - q_{out}(t)\big)dt. \qquad [7]$$

By integrating from the diastolic time instant $t_{dia}$ to the systolic time instant $t_{sys}$ we can obtain an estimate of the compliance as:

$$C = \frac{\int_{t_{dia}}^{t_{sys}} \big(q_{in}(t) - q_{out}(t)\big)dt}{P(t_{sys}) - P(t_{dias})} = \frac{\Delta V}{\Delta P}, \qquad [8]$$

with $\Delta V$ in this case being the arterial volume distension as a result of the cardiac pulse pressure $\Delta P$.

It is worth noting that it is possible to measure blood pressure and arterial flow asynchronously in time, as long as they are measured in close temporal proximity. In such a case it is advantageous to measure the arterial flow and blood pressure over a plurality of heartbeats. In fact, it may be preferred to not image the artery (i.e., determine the arterial flow via the ultrasound sensor) when a cuff usually associated with a blood pressure sensor is inflated and therefore affecting the lumen dimensions (e.g., diameter, shape).

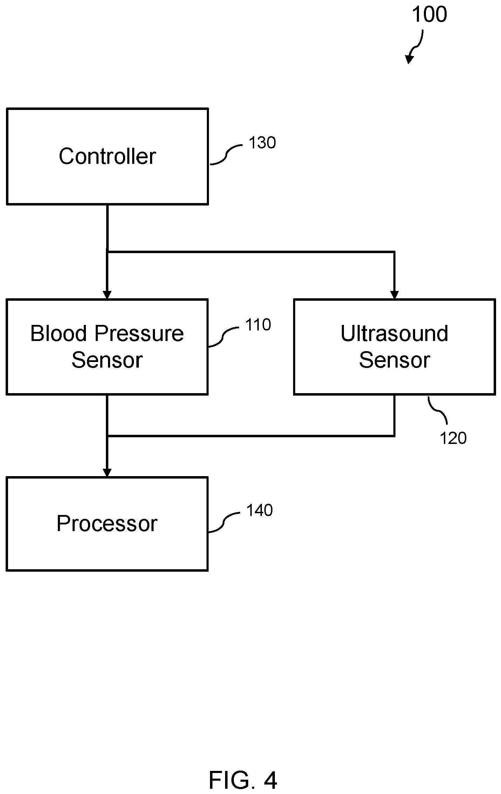

Fig.4 presents a block diagram of a system 100 for determining vascular compliance of a subject according to an aspect of the invention. That is, the system 100 is suitable for estimating how a pulse pressure relates to the underlying pulse volume of the subject responsive to a cardiac pulse. Specifically, there is provided a blood pressure sensor 110, an ultrasound sensor 120, controller 130, and processor 140. Each component may be provided in one monolithic device, or may be several different communicatively linked physical devices.

The blood pressure sensor 110 is configured to measure blood pressure of a first artery of the subject. The blood pressure may be measured by the blood pressure sensor continuously through one cardiac pulse (i.e., heart cycle or heartbeat) of the subject, or may simply measure the systolic and diastolic pressure of the artery during one cardiac pulse. In other embodiments, the blood pressure sensor may perform the above measurements over multiple cardiac pulses before producing an average, such as a phase rectified signal average (PRSA), or the arithmetic mean of the measured systolic pressures and the arithmetic mean of the measured diastolic pressures, or the arithmetic mean of the measured pulse pressures.

The ultrasound sensor 120 is configured to measure arterial flow of a second artery of the subject. That is, the ultrasound sensor is configured to measure the diameter of the second artery and/or a velocity of blood through the second artery. Each, or indeed both, measures may be used as a representative of the arterial flow of the second artery. Similarly to the above, the ultrasound sensor may measure the arterial flow (e.g., diameter and/or velocity) continuously through one cardiac pulse of the subject, or may simply sample the arterial flow at one or more parts of the cardiac pulse. Alternatively, the ultrasound sensor may perform the above measurements over multiple cardiac pulses before producing an average, such as a PRSA.

In some cases, the ultrasound sensor may be configured to operate using pulsed wave Doppler mode or color Doppler mode to measure the velocity, and to operate using M-mode or B-mode or A-mode to measure the diameter.

The first artery and the second artery are the same artery or corresponding arteries of contralateral limbs. That is, the first artery and the second artery that the ultrasound sensor and blood pressure sensor take measurements from may be a single artery or arterial branch (such as a brachial artery), or may be taken from arteries on contralateral limbs (e.g., the ultrasound sensor on one brachial artery, and the blood pressure sensor on the opposite brachial artery).

The blood pressure sensor and the ultrasound sensor may be implemented in the same device. For example, the ultrasound sensor may be integrated into an existing blood pressure sensor, such as a pressure cuff, for seamless measurement of the parameters required to determine vascular compliance.

The controller is configured to control the blood pressure sensor to measure the blood pressure. The controller is also configured to control the ultrasound sensor to measure the arterial flow. That is, the controller produces control signals to cause the blood pressure sensor and the ultrasound sensor to perform their corresponding measurements.

Furthermore, the controller may be configured to control the blood pressure sensor to measure the blood pressure, and the ultrasound sensor to measure the arterial flow simultaneously or substantially sequentially. The controller may cause the simultaneous or sequential measurement depending on the circumstances/context of the subject, and may alternate modes to provide different types of measurement.

In some cases, the controller may be configured to continually control the ultrasound sensor to measure the arterial flow. In this case, the processor may be configured to detect a change of the measured arterial flow in time meeting a predetermined condition. Then, the controller may be configured to control the blood pressure sensor to measure the blood pressure responsive to detecting the change of the arterial flow. This may provide a system to automatically detect changes in vascular compliance (and thus, likely hemodynamic parameters) without being uncomfortable or invasive.

The processor is configured to determine a pulse pressure based on the measured blood pressure. In particular, the pulse pressure may be determined by a taking a difference between the systolic and diastolic blood pressure.

Furthermore, the processor is also configured to determine a pulse volume based on the measured arterial flow. The pulse volume may be determined from the arterial flow based on any method described above.

In particular, the pulse volume may be based on a measured velocity and/or diameter. In the case that both velocity and diameter are measured, then they may be multiplied in order to determine the arterial flow of the artery. In the case that only one of the velocity or diameter are measured by the ultrasound sensor, predicted, or historic values of the velocity and/or diameter may be used alongside the actual measured value to determine the arterial flow. Integration of the arterial flow over time (providing an estimation of the blood volume through the artery in a longitudinal direction) may be performed, and from the integration a volume change of the artery, between systolic and diastolic time instants for example, determined as the pulse volume.

Then, the processor is configured to determine a vascular compliance of the subject based on the pulse pressure and the pulse volume. This may be achieved based on simply dividing the pulse pressure by the pulse volume. However, various post-processing methods may be performed on the determined pulse pressure and pulse volume in order to obtain an accurate vascular compliance determination.

In some example implementations, the processor may (additionally or alternatively) be configured to determine a time constant of exponential decay of the blood pressure based on a decay of blood pressure following systole in the continuous blood pressure signal. The processor may then determine a peripheral vascular resistance based on a mean blood pressure over the at least one heart cycle and a mean of the measured arterial flow (e.g., a mean arterial flow as determined over the at least one heart cycle). The vascular compliance of the subject can then be determined based on the time constant and the peripheral vascular resistance.

Of course, any disclosed method and means of determining vascular compliance may be employed by the system 100.

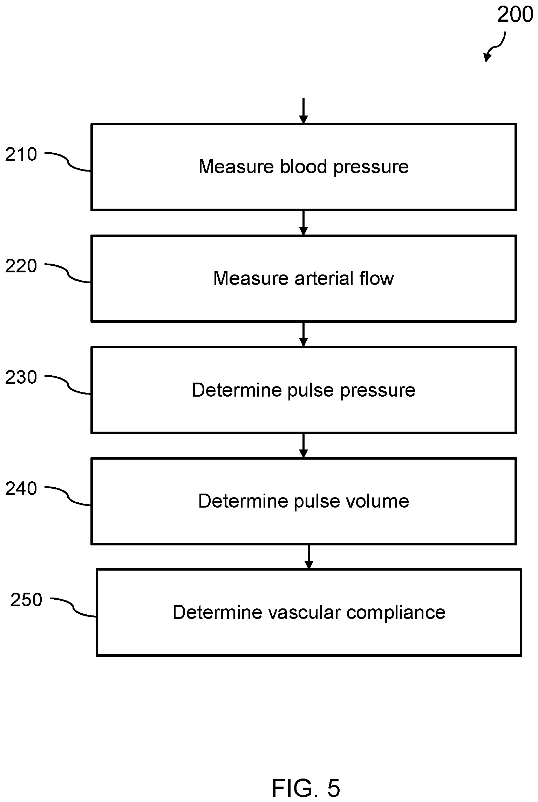

Fig.5 presents a flow diagram of a method 200 for determining vascular compliance of a subject according to another aspect of the invention.

In step 210, a blood pressure sensor is controlled to measure blood pressure of a first artery of the subject

In step 220, an ultrasound sensor is controlled to measure arterial flow of a second artery of the subject. The first artery and the second artery are the same artery or corresponding arteries of contralateral limbs.

In step 230, a pulse pressure is determined based on the measured blood pressure.

In step 240, a pulse volume is determined based on the measured arterial flow.

In step 250, vascular compliance of the subject is determined based on the pulse pressure and the pulse volume.

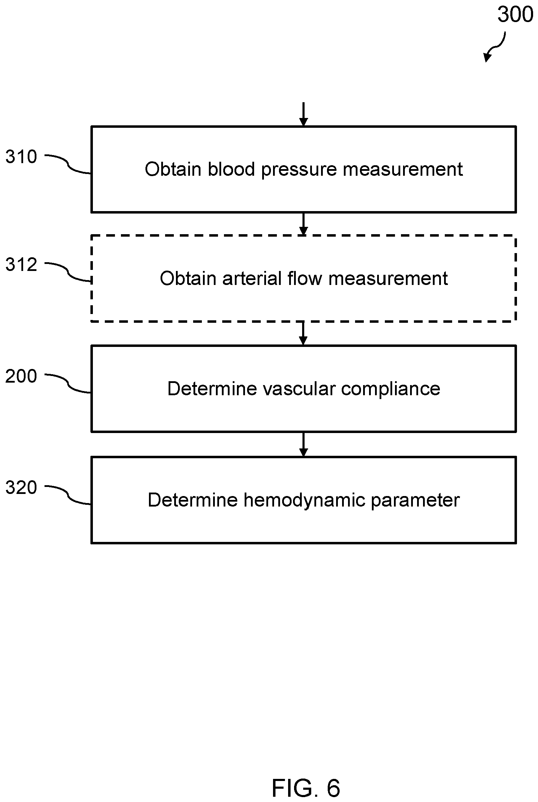

Fig.6 presents a flow diagram of a method 300 for determining a hemodynamic parameter of a subject according to a further aspect of the invention. For example, the method may be used to determine a cardiac output and/or stroke volume of the subject.

In step 310, a blood pressure measurement of a subject is obtained. The blood pressure measurement may be obtained from a blood pressure sensor, perhaps the same blood pressure sensor as in method 200 above. Alternatively, the blood pressure measurement may be a historic measurement taken previously.

In (optional) step 312, an arterial flow measurement of the subject is obtained (in addition to the blood pressure measurement). The arterial flow measurement may be obtained from an ultrasound sensor, perhaps the same ultrasound sensor as in method 200 above. Alternatively, the arterial flow measurement may be a historic measurement taken previously.

In step 200, a vascular compliance value of the subject is determined according to the method 200. This step may be performed before or after obtaining the blood pressure measurement in step 310, and may include obtaining a vascular compliance value from a database (e.g., EMR of the subject) that was determined using the method 200.

In step 320, the vascular compliance and blood pressure measurement of the subject are processed/analysed using a transfer function to determine the hemodynamic parameter. This may be achieved using any known transfer function familiar to the skilled person that uses vascular compliance and blood pressure measurements to determine hemodynamic parameters. Indeed, existing transfer functions that use demographic information as a proxy for vascular compliance may be adapted to receive the vascular compliance and output the hemodynamic parameter.

Alternatively, (if the arterial flow measurement has also been obtained) in step 320, the vascular compliance, arterial flow measurement and blood pressure measurement of the subject are processed/analysed using a transfer function to determine the hemodynamic parameter. This may be achieved using any known transfer function familiar to the skilled person that uses vascular compliance, arterial flow measurements, and blood pressure measurements to determine hemodynamic parameters. Indeed, existing transfer functions that use demographic information as a proxy for vascular compliance may be adapted to receive the vascular compliance and output the hemodynamic parameter.

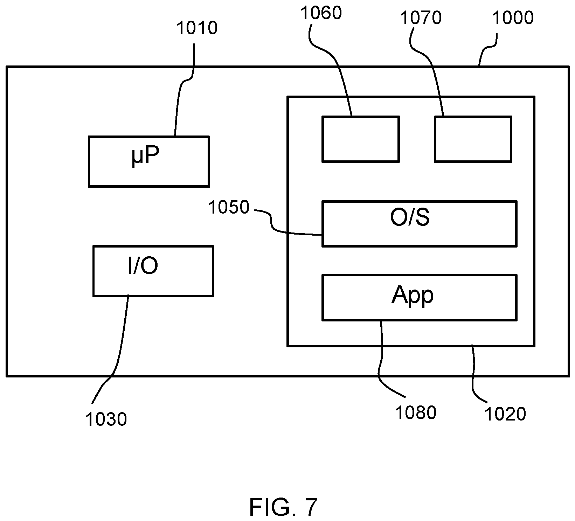

Fig.7 illustrates an example of a computer 1000 within which one or more parts of an embodiment may be employed. Various operations discussed above may utilize the capabilities of the computer 1000. For example, one or more parts of a system for acquiring an input from a user to control an interface may be incorporated in any element, module, application, and/or component discussed herein. In this regard, it is to be understood that system functional blocks can run on a single computer or may be distributed over several computers and locations (e.g. connected via internet).

The computer 1000 includes, but is not limited to, smart phones, PCs, workstations, laptops, PDAs, palm devices, servers, storages, patient monitors and the like. In many cases, the disclosed systems and methods may be implemented in a patient monitor, such as a bed side monitor. Such monitors may be used in, for example, the intensive care unit (ICU), the operating room (OR), or post-anaesthesia care unit (PACU).

Generally, in terms of hardware architecture, the computer 1000 may include one or more processors 1010, memory 1020 and one or more I/O devices 1030 that are communicatively coupled via a local interface (not shown). The local interface can be, for example but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.

The processor 1010 is a hardware device for executing software that can be stored in the memory 1020. The processor 1010 can be virtually any custom made or commercially available processor, a central processing unit (CPU), a digital signal processor (DSP), or an auxiliary processor among several processors associated with the computer 1000, and the processor 1010 may be a semiconductor-based microprocessor (in the form of a microchip) or a microprocessor.

The memory 1020 can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), solid state drive (SSD), tape, compact disc read only memory (CD-ROM), disk, diskette, cartridge, cassette or the like, etc.). Moreover, the memory 1020 may incorporate electronic, magnetic, optical, and/or other types of storage media. Note that the memory 1020 can have a distributed architecture, where various components are situated remote from one another, but can be accessed by the processor 1010.

The software in the memory 1020 may include one or more separate programs, each of which comprises an ordered listing of executable instructions for implementing logical functions. The software in the memory 1020 includes a suitable operating system (O/S) 1050, compiler 1060, source code 1070, and one or more applications 1080 in accordance with exemplary embodiments. As illustrated, the application 1080 comprises numerous functional components for implementing the features and operations of the exemplary embodiments. The application 1080 of the computer 1000 may represent various applications, computational units, logic, functional units, processes, operations, virtual entities, and/or modules in accordance with exemplary embodiments, but the application 1080 is not meant to be a limitation.

The operating system 1050 controls the execution of other computer programs, and provides scheduling, input-output control, file and data management, memory management, and communication control and related services. It is contemplated by the inventors that the application 1080 for implementing exemplary embodiments may be applicable on all commercially available operating systems.

Application 1080 may be a source program, executable program (object code), script, or any other entity comprising a set of instructions to be performed. When a source program, then the program is usually translated via a compiler (such as the compiler 1060), assembler, interpreter, or the like, which may or may not be included within the memory 1020, so as to operate properly in connection with the O/S 1050. Furthermore, the application 1080 can be written as an object oriented programming language, which has classes of data and methods, or a procedure programming language, which has routines, subroutines, and/or functions, for example but not limited to, C, C++, C#, Pascal, Python, BASIC, API calls, HTML, XHTML, XML, ASP scripts, JavaScript, FORTRAN, COBOL, Perl, Java, ADA, .NET, functional programming and the like.

The I/O devices 1030 may include input devices such as, for example but not limited to, a mouse, keyboard, scanner, microphone, camera, touch-screen, etc. Furthermore, the I/O devices 1030 may also include output devices, for example but not limited to a printer, display, etc. Finally, the I/O devices 1030 may further include devices that communicate both inputs and outputs, for instance but not limited to, a NIC or modulator/demodulator (for accessing remote devices, other files, devices, systems, or a network), a radio frequency (RF) or other transceiver, a telephonic interface, a bridge, a router, etc. The I/O devices 1030 also include components for communicating over various networks, such as the Internet or intranet.

If the computer 1000 is a PC, workstation, intelligent device or the like, the software in the memory 1020 may further include a basic input output system (BIOS) (omitted for simplicity). The BIOS is a set of essential software routines that initialize and test hardware at start-up, start the O/S 1050, and support the transfer of data among the hardware devices. The BIOS is stored in some type of read-only-memory, such as ROM, PROM, EPROM, EEPROM or the like, so that the BIOS can be executed when the computer 800 is activated.

When the computer 1000 is in operation, the processor 1010 is configured to execute software stored within the memory 1020, to communicate data to and from the memory 1020, and to generally control operations of the computer 1000 pursuant to the software. The application 1080 and the O/S 1050 are read, in whole or in part, by the processor 1010, perhaps buffered within the processor 1010, and then executed.

When the application 1080 is implemented in software it should be noted that the application 1080 can be stored on virtually any computer readable medium for use by or in connection with any computer related system or method. In the context of this document, a computer readable medium may be an electronic, magnetic, optical, or other physical device or means that can contain or store a computer program for use by or in connection with a computer related system or method.

The application 1080 can be embodied in any computer-readable medium for use by or in connection with an instruction execution system, apparatus, or device, such as a computer-based system, processor-containing system, or other system that can fetch the instructions from the instruction execution system, apparatus, or device and execute the instructions. In the context of this document, a "computer-readable medium" can be any means that can store, communicate, propagate, or transport the program for use by or in connection with the instruction execution system, apparatus, or device. The computer readable medium can be, for example but not limited to, an electronic, magnetic, optical, electromagnetic, infrared, or semiconductor system, apparatus, device, or propagation medium.

The methods described in relation to Fig.5 and 6, and the systems described in relation to Fig.4, may be implemented in hardware or software, or a mixture of both (for example, as firmware running on a hardware device). To the extent that an embodiment is implemented partly or wholly in software, the functional steps illustrated in the process flowcharts may be performed by suitably programmed physical computing devices, such as one or more central processing units (CPUs) or graphics processing units (GPUs). Each process - and its individual component steps as illustrated in the flowcharts - may be performed by the same or different computing devices. According to embodiments, a computer-readable storage medium stores a computer program comprising computer program code configured to cause one or more physical computing devices to carry out an encoding or decoding method as described above when the program is run on the one or more physical computing devices.

Storage media may include volatile and non-volatile computer memory such as RAM, PROM, EPROM, EEPROM, and SSD, optical discs (like CD, DVD, BD), magnetic storage media (like hard discs and tapes). Various storage media may be fixed within a computing device or may be transportable, such that the one or more programs stored thereon can be loaded into a processor.

To the extent that an embodiment is implemented partly or wholly in hardware, the blocks shown in the block diagram of Fig.4 may be separate physical components, or logical subdivisions of single physical components, or may be all implemented in an integrated manner in one physical component. The functions of one block shown in the drawings may be divided between multiple components in an implementation, or the functions of multiple blocks shown in the drawings may be combined in single components in an implementation. Hardware components suitable for use in embodiments of the present invention include, but are not limited to, conventional microprocessors, application specific integrated circuits (ASICs), and field-programmable gate arrays (FPGAs). One or more blocks may be implemented as a combination of dedicated hardware to perform some functions and one or more programmed microprocessors and associated circuitry to perform other functions.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

The flowchart and block diagrams in the Figures illustrate the architecture, functionality, and operation of possible implementations of systems, methods, and computer program products according to various embodiments of the present invention. In this regard, each block in the flowchart or block diagrams may represent a module, segment, or portion of instructions, which comprises one or more executable instructions for implementing the specified logical function(s). In some alternative implementations, the functions noted in the block may occur out of the order noted in the figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions.

**Claims**

1. A system (100) for determining vascular compliance of a subject, comprising:

   a blood pressure sensor (110) configured to measure blood pressure of a first artery of the subject;
   an ultrasound sensor (120) configured to measure arterial flow of a second artery of the subject, wherein the first artery and the second artery are the same artery or corresponding arteries of contralateral limbs;
   a controller (130) configured to:

   control the blood pressure sensor to measure the blood pressure;
   control the ultrasound sensor to measure the arterial flow; and

   a processor (140) configured to:

   determine a pulse pressure based on the measured blood pressure;
   determine a pulse volume based on the measured arterial flow; and
   determine a vascular compliance of the subject based on the pulse pressure and the pulse volume.

2. The system of claim 1, wherein the controller (130) is configured to control the blood pressure sensor (110) to measure a systolic pressure and a diastolic pressure, and wherein the processor (140) is configured to determine the pulse pressure based on the systolic pressure and the diastolic pressure.

3. The system of claim 1 or 2, wherein the ultrasound sensor (120) is configured to measure a velocity of blood through the second artery and a diameter of the second artery, and wherein the controller (130) controls the ultrasound sensor to measure the velocity and the diameter, and wherein the processor (140) is configured to determine the pulse volume based on the velocity and the diameter.

4. The system of claim 3, wherein the ultrasound sensor (120) is configured to operate using pulsed wave Doppler mode or color Doppler mode to measure the velocity, and to operate using M-mode, B-mode or A-mode to measure the diameter.

5. The system of claim 1 or 2, wherein the ultrasound sensor (120) is configured to measure a diameter of the second artery, and wherein the controller (130) controls the ultrasound sensor to measure the diameter, and wherein the processor (140) is configured to determine the pulse volume based on the diameter.

6. The system of claim 1 or 2, wherein the ultrasound sensor (120) is configured to measure a velocity of blood through the second artery, and wherein the controller (130) controls the ultrasound sensor to measure the velocity, and wherein the processor (140) is configured to determine the pulse volume based on the velocity.

7. The system of any of claims 1-6, wherein the controller (130) is further configured to control the blood pressure sensor to measure a continuous blood pressure signal over at least one heart cycle; and
   wherein the processor (140) is further configured to:

   determine a time constant of exponential decay of the blood pressure based on a decay of blood pressure following systole in the continuous blood pressure signal;
   determine a peripheral vascular resistance based on a mean blood pressure over the at least one heart cycle and a mean of the measured arterial flow; and
   determine the vascular compliance of the subject based on the time constant and the peripheral vascular resistance.

8. The system of any of claims 1-7, wherein the first artery and the second artery are the same artery, and wherein the blood pressure sensor (110) is configured to measure blood pressure of the first artery of the subject at a first location,

   wherein the ultrasound sensor (120) is configured to measure arterial flow of the second artery in two longitudinally separated locations of the second artery, the first location between the two longitudinally separated locations, and
   wherein the processor (140) is configured to determine the vascular compliance of the subject further based on the measured arterial flow in the two longitudinally separated locations.

9. The system of any of claims 1-8, wherein the controller (130) is configured to:

   control the blood pressure sensor (110) to measure the blood pressure for a first time period;
   control the ultrasound sensor (120) to measure the arterial flow for the first time period;
   control the blood pressure sensor to measure the blood pressure for a second time period; and
   control the ultrasound sensor to measure the arterial flow for the second time period, wherein the second time period and first time period are sequential, and
   wherein the processor (140) is configured to:

      determine the pulse pressure based on a difference between the measured blood pressure in the first time period and the measured blood pressure in the second time period; and
      determine a pulse volume based on a difference between the measured arterial flow in the first time period and the measured arterial flow in the second time period.

10. The system of any of claims 1-9, wherein the controller (130) is configured to control the blood pressure sensor to measure the blood pressure over a plurality of heart cycles, and wherein the processor (140) is configured to determine the pulse pressure based on an average of the measured blood pressure over the plurality of heart cycles.

11. The system of any of claims 1-10, wherein the controller (130) is configured to control the ultrasound sensor (120) to measure the arterial flow over a plurality of heartbeats, and wherein the processor (140) is configured to determine the pulse volume based on an average of the arterial flow over the plurality of heart beats.

12. The system of any of claims 1-11, wherein the controller (130) is configured to continually control the ultrasound sensor (120) to measure the arterial flow,

   wherein the processor (140) is configured to detect a change of the measured arterial flow in time, and
   wherein the controller is configured to control the blood pressure sensor (110) to measure the blood pressure responsive to detecting the change of the arterial flow.

13. A method (200) of determining vascular compliance of a subject, comprising:

controlling (210) a blood pressure sensor to measure blood pressure of a first artery of the subject;
controlling (220) an ultrasound sensor to measure arterial flow of a second artery of the subject, wherein the first artery and the second artery are the same artery or corresponding arteries of contralateral limbs;
determining (230) a pulse pressure based on the measured blood pressure;
determining (240) a pulse volume based on the measured arterial flow; and
determining (250) a vascular compliance of the subject based on the pulse pressure and the pulse volume.

14. A method (300) for determining hemodynamic parameters of a subject, comprising:

obtaining (310) a blood pressure measurement of a subject, and optionally obtaining (312) an arterial flow measurement of the subject;
determining (200) vascular compliance of the subject according to claim 13; and
processing (320) the vascular compliance and blood pressure measurement of the subject using a transfer function to determine the hemodynamic parameter, and optionally further processing the arterial flow measurement of the subject using the transfer function to determine the hemodynamic parameter.

15. A computer program comprising computer program code means adapted, when said computer program is run on a computer, to implement the method of any of claim 13 or 14.

FIG. 1

FIG. 2

FIG. 3

100

```
┌─────────────────┐
│                 │
│   Controller    │ ⌐ 130
│                 │
└────────┬────────┘
         │
         ├──────────────────────┐
         │                      │
         ▼                      ▼
┌─────────────────┐    ┌─────────────────┐
│ Blood Pressure  │⌐110│   Ultrasound    │
│     Sensor      │    │     Sensor      │
└────────┬────────┘    └────────┬────────┘
         │                      │  ⌐ 120
         ├──────────────────────┘
         ▼
┌─────────────────┐
│                 │
│    Processor    │ ⌐ 140
│                 │
└─────────────────┘
```

FIG. 4

200

210 — Measure blood pressure

220 — Measure arterial flow

230 — Determine pulse pressure

240 — Determine pulse volume

250 — Determine vascular compliance

FIG. 5

300

310 — Obtain blood pressure measurement

312 — Obtain arterial flow measurement

200 — Determine vascular compliance

320 — Determine hemodynamic parameter

FIG. 6

FIG. 7

Europäisches
Patentamt

European
Patent Office

Office européen
des brevets

# EUROPEAN SEARCH REPORT

Application Number

EP 23 19 6543

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2015/351703 A1 (PHILLIPS ROBERT ALLAN [AU] ET AL) 10 December 2015 (2015-12-10) * abstract * * figures 1-8 * * paragraph [0016] - paragraph [0210] * ----- | 1-15 | INV. A61B8/04 A61B8/06 A61B8/08 A61B5/02 A61B8/00 |
| A | US 2011/066048 A1 (TSUJI TOSHIO [JP] ET AL) 17 March 2011 (2011-03-17) * abstract * * figures 1-17 * * paragraph [0021] - paragraph [0108] * ----- | 1-15 | |
| A | US 2021/100523 A1 (JIANG XIAOYUE [US] ET AL) 8 April 2021 (2021-04-08) * abstract * * figures 1-14 * * paragraph [0003] - paragraph [0096] * ----- | 1-15 | |
| | | | TECHNICAL FIELDS SEARCHED (IPC) A61B |

The present search report has been drawn up for all claims

| Place of search | Date of completion of the search | Examiner |
|---|---|---|
| Munich | 31 January 2024 | Moehrs, Sascha |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons
............................................................................
& : member of the same patent family, corresponding
   document

EPO FORM 1503 03.82 (P04C01)

## ANNEX TO THE EUROPEAN SEARCH REPORT
## ON EUROPEAN PATENT APPLICATION NO.

**EP 23 19 6543**

This annex lists the patent family members relating to the patent documents cited in the above-mentioned European search report.
The members are as contained in the European Patent Office EDP file on
The European Patent Office is in no way liable for these particulars which are merely given for the purpose of information.

**31-01-2024**

| Patent document cited in search report | Publication date | Patent family member(s) | Publication date |
|---|---|---|---|
| US 2015351703 A1 | 10-12-2015 | EP 2943126 A1 | 18-11-2015 |
| | | US 2015351703 A1 | 10-12-2015 |
| | | WO 2014107769 A1 | 17-07-2014 |
| US 2011066048 A1 | 17-03-2011 | EP 2294976 A1 | 16-03-2011 |
| | | JP 5399832 B2 | 29-01-2014 |
| | | JP 2011056200 A | 24-03-2011 |
| | | US 2011066048 A1 | 17-03-2011 |
| US 2021100523 A1 | 08-04-2021 | NONE | |

EPO FORM P0459

For more details about this annex : see Official Journal of the European Patent Office, No. 12/82